# EUROPÄISCHE PATENTANMELDUNG

(11) **EP 3 603 560 A1**
(43) Veröffentlichungstag der Anmeldung: **05.02.2020**
(21) Anmeldenummer: 18186562.7
(22) Anmeldetag: 31.07.2018
(51) Int. Cl.: A61B 50/36, B65F 1/10, A61B 50/00

(54) **AUFNAHMEVORRICHTUNG ZUR AUFNAHME VON IM MEDIZINISCHEN UMFELD ANFALLENDEM LÄNGLICHEM ABFALL**

(71) Anmelder: Siemens Healthcare GmbH, 91052 Erlangen (DE)
(72) Erfinder: Die Erfindernennung liegt noch nicht vor

(57) **Zusammenfassung**

Für eine hygienische und sichere Arbeitsumgebung im interventionellen Operationsraum ist ein Aufnahmevorrichtung zur Aufnahme von im medizinischen Umfeld anfallendem länglichem Abfall, aufweisend einen Behälter mit einer Einlassvorrichtung, welche Einlassvorrichtung derartig ausgebildet ist, dass längliche Gegenstände automatisch von außen ins Innere des Behälters transportiert werden, vorgesehen

## Beschreibung

Die Erfindung betrifft eine Aufnahmevorrichtung zur Aufnahme von im medizinischen Umfeld anfallendem länglichem Abfall gemäß dem Patentanspruch 1.

Während medizinischer Eingriffe im interventionellen Umfeld (z.B. Operationsraum) entsteht häufig eine größere Menge an Abfall. So werden zum Beispiel benutzte Führungsdrähte, Katheter und Schläuche routinemäßig weggeworfen. Im Allgemeinen werden derartige längliche Gegenstände in einen offenen Abfallbehälter geworfen. Aufgrund ihrer Länge und Steifheit hängen sie häufig aus dem Abfallbehälter heraus oder fallen auf den Fußboden des Untersuchungsraumes. Dies ist nicht nur unhygienisch, sondern auch gefährlich, da Personen dadurch gestört oder sogar zu Fall gebracht werden können, wenn sie darüber stolpern. Die Lösung könnte sein, einen möglich großen Abfallbehälter zu verwenden, dieser blockiert aber dann erst recht notwendige Zugangswege. Die Person, die die Abfälle wegwirft, trägt im Allgemeinen sterile Handschuhe und sollte den Abfallbehälter nicht berühren, um nicht jedes Mal die Handschuhe wechseln zu müssen.

Es ist Aufgabe der vorliegenden Erfindung, eine Vorrichtung bereitzustellen, welche das hygienische Wegwerfen von benutzten Führungsdrähten und Kathetern ermöglicht.

Die Aufgabe wird erfindungsgemäß gelöst durch eine Aufnahmevorrichtung zur Aufnahme von im medizinischen Umfeld anfallendem länglichem Abfall gemäß dem Patentanspruch 1. Vorteilhafte Ausgestaltungen der Erfindung sind Gegenstand der zugehörigen Unteransprüche.

Die erfindungsgemäße Aufnahmevorrichtung zur Aufnahme von im medizinischen Umfeld anfallendem länglichem Abfall, aufweisend einen Behälter mit einer Einlassvorrichtung, welche derartig ausgebildet ist, dass längliche Gegenstände, wie z.B. Führungsdrähte, Katheter und Schläuche, automatisch von außen ins Innere des Behälters transportiert werden, gewährleistet, dass der länglich geformte Abfall, z.B. die gebrauchten Führungsdrähte, Katheter oder Schläuche, auf einfache Weise, sicher und hygienisch ins Innere des Behälters transportiert werden. Dadurch wird verhindert, dass der Abfall aus dem Behälter herausfällt und dadurch ein Sicherheitsrisiko für Personen oder ein Hindernis für Geräte und Zugangswege darstellt. Auch für die Hygiene im Operationsraum ist die erfindungsgemäße Aufnahmevorrichtung von großem Vorteil, da keine der Personen im Operationsraum mit sterilen Handschuhen den Abfall nochmals anfassen muss oder sogar den Abfallbehälter oder den Boden berühren muss.

Nach einer Ausgestaltung der Erfindung weist die Einlassvorrichtung zumindest zwei um je eine Achse rotierbare, gegenläufig antreibbare Walzen auf, und wobei die Walzen derart angeordnet sind, dass zwischen ihnen ein an die Gegenstände anpassbarer Einlass angeordnet ist. Die Walzen sind dabei möglichst immer paarweise vorhanden, um ein gegenläufiges aufeinander zu rotieren zu ermöglichen. Hierdurch kann der längliche Abfall in den Einlass gezogen und von dort ins Innere des Behälters transportiert werden. Die Walzen können durch einen elektrischen Antrieb motorisch bewegt werden.

In vorteilhafter Weise für eine besonders einfache Aufnahme des länglichen Abfalls sind die jeweils zwei gegenläufig antreibbaren Walzen flexibel miteinander verbunden und weisen als Einlass eine durch Krafteinwirkung öffenbare und/oder veränderbare Einlassöffnung auf. Die Einlassöffnung ist dadurch an verschiedene Dicken des länglichen Abfalls anpassbar, so dass für alle möglichen Führungsdrähte, Katheter oder Schläuche eine einfache Aufnahme möglich ist und keine Abfallteile wieder aus dem Behälter herausfallen können. Durch die jeweilige Ausdehnung des Abfalls wird eine entsprechende Krafteinwirkung erzeugt, welche die Walzen so weit auseinander drückt, dass eine passende Einlassöffnung entsteht. In diese Einlassöffnung wird der Abfall durch die gegenläufige Rotation der Walzen gezogen und ins Innere des Behälters transportiert.

Nach einer weiteren Ausgestaltung der Erfindung sind die jeweils zwei gegenläufig antreibbaren Walzen mittels einer Federvorrichtung miteinander verbunden. Die Federvorrichtung kann den Abstand zwischen den Walzen verändern, so dass eine besonders flexible Anpassung an verschiedene Ausdehnungen bzw. Durchmesser des länglichen Abfalls gewährleistet ist. Die Federvorrichtung kann z.B. aus einer oder mehreren Federn bestehen.

Nach einer weiteren Ausgestaltung der Erfindung stoßen die jeweils zwei gegenläufig antreibbaren Walzen in einem Ruhemodus ohne Krafteinwirkung bündig aneinander. Hierdurch ist im Ruhezustand, also wenn kein Abfall eingezogen werden soll und/oder die Walzen sich nicht bewegen, der Behälter geschlossen.

Nach einer weiteren Ausgestaltung der Erfindung weist die Aufnahmevorrichtung zumindest ein Ventil innerhalb des Behälters auf, welches angrenzend an die Einlassöffnung angeordnet ist. Hierdurch wird noch zusätzlich zu der schließbaren Einlassöffnung sichergestellt, dass kein Abfall wieder aus dem Behälter herausfallen kann oder durch Hängenbleiben an den Walzen wieder herausbefördert werden kann. Insbesondere weist das zumindest eine Ventil mindestens ein Klappensegel auf. Ein bzw. mehrere derartige Klappensegel sind ebenso wie die Einlassöffnung nur in eine Richtung passierbar und verhindern somit ein Austreten des Abfalls aus dem Behälter.

Nach einer weiteren Ausgestaltung der Erfindung ist innerhalb des Behälters eine Zerkleinerungsvorrichtung angeordnet. Hierdurch kann der Abfall in kleine Teile zerlegt und somit später einfacher entsorgt werden.

Nach einer weiteren Ausgestaltung der Erfindung weist die Aufnahmevorrichtung Sensoren, insbesondere Näherungssensoren zur Detektion eines sich an die Einlassvorrichtung annähernden Objekts oder Berührungssensoren zur Detektion einer Berührung, und eine Triggervorrichtung zur Aktivierung der Walzen im Falle einer Annäherung oder Berührung auf. Auf diese Weise kann ein sich näherndes oder berührendes Objekt, also Abfall, detektiert werden und bei Überschreiten eines Schwellwertes der Annäherung oder bei Berührung werden die Walzen motorisch in Bewegung versetzt. Alternativ können die Walzen auch durch einen Schalter (z.B. Fußschalter) in Bewegung gesetzt werden oder kontinuierlich bewegt werden. Hierdurch wird gewährleistet, dass medizinisches Personal mit sterilen Handschuhen den Behälter nicht anfassen muss.

Nach einer weiteren Ausgestaltung der Erfindung sind die zumindest zwei Walzen aus einem abwaschbaren Material ausgebildet oder mit zumindest einer Lage aus wasserfestem, entfernbarem Einwegmaterial umhüllt. Die Walzen können z.B. aus einem schnell reinigbaren Kunststoff gebildet sein oder von einer oder einer Vielzahl von Lagen von Polyethylenefolie umhüllt sein. Nach einem Eingriff werden dann die Walzen entweder einzeln gereinigt oder es wird jeweils eine Lage des Einwegmaterials entfernt. Es ist vorteilhaft, die Walzen derart baulich anzuordnen, dass sie mit einfachen Handgriffen entfernbar sind.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:
- FIG 1: eine Schnittansicht einer erfindungsgemäßen Aufnahmevorrichtung zur Aufnahme von im medizinischen Umfeld anfallendem länglichem Abfall;
- FIG 2: eine Schnittansicht gemäße FIG 1 mit geöffneter Einlassöffnung.

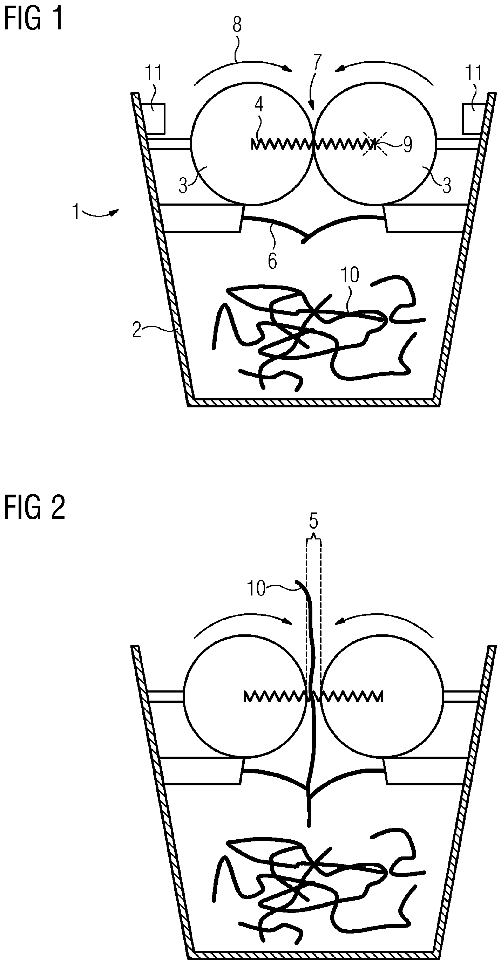

In der FIG 1 ist eine Aufnahmevorrichtung 1 zur Aufnahme von im medizinischen Umfeld anfallendem länglichem Abfall gezeigt. Derartiger länglicher Abfall, zum Beispiel gebrauchte Führungsdrähte, Katheter oder Schläuche, fällt insbesondere bei interventionellen Eingriffen im Operationsraum an und muss möglichst hygienisch und sicher verstaut werden. Die Aufnahmevorrichtung weist einen Behälter 2 sowie eine Einlassvorrichtung 3; 4; 6; 9 auf. Die Einlassvorrichtung weist z.B. zwei Walzen 3 auf, welche um je eine Achse 9 motorisch angetrieben (Antrieb ist nicht explizit gezeigt) rotiert werden können, wobei sie zur Aufnahme von länglichem Abfall 10 in gegenläufiger Richtung 8 aufeinander zu bewegt werden. Die beiden Walzen 3 werden durch eine Federvorrichtung 4 zusammengehalten. Mittels der Federvorrichtung 4 (z.B. eine Feder) können durch Krafteinwirkung die beiden Walzen auseinandergedrückt werden, so dass eine Einlassöffnung 5 entsteht.

Die Einlassöffnung ist zum Beispiel schlitzförmig und flexibel verstellbar und passt sich in ihrer Breite an die z.B. durch die Dicke des länglichen Abfalls 10 ausgeübte Kraft an - siehe FIG 2. Durch die Walzen 3 bzw. deren aufeinander gerichtete Rotation wird der längliche Abfall 10 automatisch ins Innere des Behälters 2 gezogen. Im Ruhemodus ohne Krafteinwirkung, also zum Beispiel wenn kein Abfall sich im Bereich der Walzen 3 befindet oder wenn die Walzen nicht aktiviert sind, stoßen die jeweils zwei Walzen 3 bündig aneinander und verschließen somit die Einlassöffnung 5.

An die Walzen 3 schließt sich im Inneren des Behälters 2 ein Ventil mit zwei biegbaren Klappensegeln 6 an, welche den länglichen Abfall 10 weiter ins Innere des Behälters 2 führen. Da das Ventil nur in eine Richtung passierbar ist, wird hierdurch zusätzlich verhindert, dass der längliche Abfall 10 sich um die Walzen 3 wickelt oder aus dem Inneren des Behälters 2 entkommt. Das Ventil kann auch mehr als zwei Klappensegel aufweisen oder anders geformt sein, wichtig ist, dass es nur in eine Richtung durchgängig ist. Anstelle des Ventils oder zusätzlich dazu kann im Inneren des Behälters 2 auch eine Zerkleinerungsvorrichtung angeordnet sein, welche den länglichen Abfall 10 in kleine Teile zerlegt, so dass er einfacher entsorgt werden kann.

Anstelle der zwei Walzen 3 können auch vier oder mehr Walzen vorgesehen sein, wobei die Walzen bevorzugt paarweise angeordnet sind, um eine gegenläufige Rotation mit Einzug des Abfalls zu gewährleisten. Es kann auch eine Vielzahl von Walzen angeordnet sein.

An der Aufnahmevorrichtung können ein oder mehrere Sensoren 11, z.B. Näherungssensoren oder Berührungssensoren, angeordnet sein, welche die Annäherung oder Berührung von Objekten detektieren. Wird eine vorbestimmte Distanz unterschritten oder eine Berührung detektiert, so kann dadurch getriggert der Antrieb der Walzen aktiviert werden und die Walzen in Bewegung gesetzt werden. Alternativ können die Walzen auch durch einen Schalter, z.B. Fußschalter aktiviert werden oder kontinuierlich in Betrieb sein.

Da die gebrauchten Führungsdrähte und Katheter häufig mit Körperflüssigkeiten verunreinigt sind, ist es vorteilhaft, die Walzen nach dem Betrieb zu reinigen. Aus diesem Grund können diese zum Beispiel aus einem leicht abwaschbaren/ desinfizierbaren Kunststoff ausgebildet sein und außerdem leicht aus der Aufnahmevorrichtung entfernbar und wieder einsetzbar sein. Alternativ können die Walzen auch zum Beispiel mit mehreren Lagen von Einwegmaterial umgeben sein (z.B. Polyethylenfolie) und nach jedem Gebrauch eine Lage davon entfernt werden.

Die Erfindung lässt sich in folgender Weise kurz zusammenfassen: Für eine hygienische und sichere Arbeitsumgebung im interventionellen Umfeld (z.B. Operationsraum) ist ein Aufnahmevorrichtung zur Aufnahme von im medizinischen Umfeld anfallendem länglichem Abfall vorgesehen, aufweisend einen Behälter mit einer Einlassvorrichtung, welche Einlassvorrichtung derartig ausgebildet ist, dass längliche Gegenstände automatisch von außen ins Innere des Behälters transportiert werden. Der längliche Abfall, z.B. die gebrauchten Führungsdrähte und Katheter, können so auf einfache Weise, sicher und hygienisch ins Innere des Behälters transportiert werden. Dadurch wird verhindert, dass der Abfall aus dem Behälter herausfällt und dadurch ein Sicherheitsrisiko darstellt. Auch für die Hygiene im Operationsraum ist dies von Vorteil, da niemand den Abfall nochmals mit den Händen anfassen muss oder sogar den Abfallbehälter oder den Boden berühren muss.

## Patentansprüche

1. Aufnahmevorrichtung (1) zur Aufnahme von im medizinischen Umfeld anfallendem länglichem Abfall, aufweisend einen Behälter (2) mit einer Einlassvorrichtung, welche Einlassvorrichtung derartig ausgebildet ist, dass längliche Gegenstände automatisch von außen ins Innere des Behälters (2) transportiert werden.

2. Aufnahmevorrichtung nach Anspruch 1, wobei die Einlassvorrichtung zumindest zwei um je eine Achse (9) rotierbare, gegenläufig antreibbare Walzen (3) aufweist, und wobei die Walzen (3) derart angeordnet sind, dass zwischen ihnen ein an die Gegenstände anpassbarer Einlass angeordnet ist.

3. Aufnahmevorrichtung nach Anspruch 2, wobei die jeweils zwei gegenläufig antreibbaren Walzen (3) flexibel miteinander verbunden sind und als Einlass eine durch Krafteinwirkung öffenbare und/oder veränderbare Einlassöffnung (5) aufweisen.

4. Aufnahmevorrichtung nach einem der Ansprüche 2 oder 3, wobei die jeweils zwei gegenläufig antreibbaren Walzen (3) mittels einer Federvorrichtung (4) miteinander verbunden sind.

5. Aufnahmevorrichtung nach einem der Ansprüche 2 bis 4, wobei die jeweils zwei gegenläufig antreibbaren Walzen (3) in einem Ruhemodus ohne Krafteinwirkung bündig aneinanderstoßen.

6. Aufnahmevorrichtung nach einem der vorangehenden Ansprüche, wobei die Aufnahmevorrichtung (1) zumindest ein Ventil innerhalb des Behälters (2) aufweist, welches angrenzend an die Einlassöffnung (5) angeordnet ist.

7. Aufnahmevorrichtung nach einem der vorangehenden Ansprüche, wobei das zumindest eine Ventil mindestens ein Klappensegel (6) aufweist.

8. Aufnahmevorrichtung nach einem der vorangehenden Ansprüche, bei welcher innerhalb des Behälters (2) eine Zerkleinerungsvorrichtung angeordnet ist.

9. Aufnahmevorrichtung nach einem der vorangehenden Ansprüche, aufweisend Sensoren (11), insbesondere Näherungssensoren zur Detektion eines sich an die Einlassvorrichtung annähernden Objekts oder Berührungssensoren zur Detektion einer Berührung, und eine Triggervorrichtung zur Aktivierung der Walzen (3) im Falle einer Annäherung oder Berührung.

10. Aufnahmevorrichtung nach einem der vorangehenden Ansprüche, wobei die zumindest zwei Walzen (3) aus einem abwaschbaren Material ausgebildet sind oder mit zumindest einer Lage aus wasserfestem, entfernbarem Einwegmaterial umhüllt sind.
